# EUROPEAN PATENT APPLICATION

(11) **EP 3 170 549 A1**
(43) Date of publication of application: **24.05.2017**
(21) Application number: 15382571.6
(22) Date of filing: 17.11.2015
(51) Int. Cl.: B01F 3/04, B01F 7/16, B01F 13/08, B01F 15/02, A61K 9/12

(54) **METHODS, DEVICES, SYSTEMS AND KITS FOR PREPARING COMPOSITIONS FOR CARE AND REPAIR OF VARICOSE VEINS**

(71) Applicant: Roche Rebollo, Enrique, 08034 Barcelona (ES)
(72) Inventor: ROCHE REBOLLO, Enrique, 08034 BARCELONA (ES); LLUSÀ MELÉNDEZ, Guiu, 08018 BARCELONA (ES)
(74) Representative: ZBM Patents - Zea, Barlocci & Markvardsen

(57) **Abstract**

The present disclosure relates to a container (19) for the production of a foamed sclerosant composition, to kits and systems including such a container (19), to methods for preparing a foamed sclerosant composition using such containers, and to foamed sclerosant compositions obtainable by such methods. In an aspect, the container (19) comprises a sealed sterile container body having one or more sidewalls extending between a top and a bottom of the container body and defining a foaming space (21). The container (19) further comprises a mixing element disposed in the container body. The container (19) contains a previously introduced gas and liquid sclerosant composition. The mixing element is configured to be operatively coupled with a rotatable actuator without the actuator reaching the foaming space (21). A medical professional may select the appropriate quantity and concentration for every treatment.

## Description

The present disclosure is related to the technical field of vascular medicine, more particularly to the field of treatments for varicose veins and other vascular problems such as e.g. spider veins and / or haemorrhoids.

Specifically, the present disclosure relates to devices, systems, kits and methods for obtaining a foam, which can be used for the treatment of the affected veins. The present disclosure further relates to the composition of the foam itself obtained by any of the procedures described throughout the present disclosure, as well as the use of the devices, systems and kits in order to treat varicose veins and other vascular problems such as e.g. spider veins and/or haemorrhoids.

### BACKGROUND

Varicose veins occur when the venous valves (which prevent the backflow of blood) do not work properly. As a result, the vein walls are weakened, and they can become deformed and dilated. Due to the fact that the valves do not work properly, the blood may recirculate and short-circuits may be created. Subsequently, the veins may become progressively dilated. In this way, the varicose veins can become more visible, and can be full of bends and become more voluminous. The evolution of this pathology may lead to consequences beyond the cosmetic ones, such as discoloration of the skin, pain and swelling of the extremities due to the effect of the venous hypertension.

According to the Spanish Society of Angiology and Vascular Surgery (SEACV) in Spain, varicose veins affect from 30 to 33 % of the adult population in industrialized countries.

The veins most commonly affected are those located in the legs, although varicose veins may occur interiorly as well: e.g. varicose veins in the esophagus, around organs located in the pelvis (pelvic and ovarian varicose veins) or at or near the most distal part of the digestive tube, near the anus (haemorrhoids).

Nowadays, there are many different treatments and/or strategies in order to mitigate or eliminate these problems. Among them, we can find surgical methods. These surgical methods are related to the surgical extraction of the affected veins using classical surgery. This technique has been employed for over 100 years. This technique is based on making different skin incisions with subsequent clamping, ligation and stripping of the venous segments affected. This technique is performed under spinal anesthesia (infiltration of the spinal space) in order to obtain the anesthesia of both limbs, as well as with local or nerve block anesthesia to anesthetize more limited areas.

In the late 1990's, there was an important advance regarding the treatment of this disease because the varicose veins were treated with less invasive techniques such as the endovenous techniques. Among these new methods, it is important to highlight intravenous procedures that apply heat through a catheter (endolaser or radio frequency systems).

These systems were able to reduce injuries due to the fact that the methods are usually performed under ultrasound guidance. The effect to the veins was produced by the release of heat or electricity from inside the vein, thus an internal injury into the vein may be produced. This way, a thrombosis of the veins was achieved.

The other technique developed was sclerotherapy by injection of a sclerosing foam. This technique (compared to the "Endolaser" or radiofrequency therapies) is even less aggressive, less painful, and needs no anesthesia (R Van den Bos et al. Endovenous therapies varicosites of lower extremity. A meta-analysis. J Vasc Surg 2009 Jan; 49 (1): 230- 9).

In summary, in the late 1990's and early 2000 a trend was found to minimize the aggressiveness of varicose veins treatments. Of all the new emerged techniques, sclerotherapy seems to be the less invasive and the one which can be applied to practically any type of varicose veins.

For this reason, in recent years, this procedure has been developed a lot and there has been a growing interest from the scientific community in order to obtain methods suitable for the manufacture of a foam in a safe and convenient way.

Sclerotherapy involves the injection of a liquid with the ability to irritate the vascular endothelium (a thin layer or lining inside the vein that is in contact with the bloodstream).

This drug or liquid medicine can become a foam when shaken. The products internationally approved for this use are lauromacrogol (also known named as polidocanol and commercially available as etoxiesclerol®), and sodium tetradecyl sulfate.

The advantage of using such a product as a foam is based on the enhancement of its effect due to the larger contact surface with the endothelial wall. The larger contact surface offers the possibility of dose reduction. Also the visibility of the drug as a foam using ultrasounds through the ultrasound scanner is improved (Schadeck M, Allaert FA. Duplex scanning in the mechanism of the sclerotherapy: Importance of the spasm. Phlebology 1995; Suppl1: 574-576).

The effect produced by the foam on the endothelium involves the injury of the cell layer of the vein, whereby thrombosis of its content is produced. Later, this vein suffers a fibrosis process (retraction and disposal) and the vein eventually may disappear after several months.

This process can be faster or slower depending on the size of the vein or the potency of the varicose agent. Therefore, it is sometimes necessary to apply several sessions on the vein.

Although there are several methods in order to eliminate or remove varicose veins, the less aggressive and less disabling treatment so far, and the one which can be used for a wide variety of pathologies is the ultrasound-guided Foam Sclerotherapy using polidocanol or other sclerosing agents. Sclerotherapy is the least invasive treatment of varicose veins known today as it can be performed in a physician's office and in a completely ambulatory way. Therefore, the present disclosure is focused on the use of this technique.

In 1995, Dr. Juan Cabrera presented the results of the application of a foam that he had developed with his son, the pharmacist Juan Cabrera (Cabrera J. et al. "Treatment of Varicose Long Saphenous Veins of Sclerosants in Microfoam Form With: Long -term Outcomes". Phlebology (2000) 15; 19-23). This foam was characterized by its density and high solubility thanks to the use of a mixture of physiological gases.

Furthermore, he managed to achieve a type of foam with very small and uniform bubble size, thanks to his method of using a mixer. By using a mixture of physiological gases, the foam had greater security and stability. This foam was called "*microfoam*" due to the small bubble size, uniformity and stability.

Shortly after, the maximum popularization of the sclerosing foam use came from Lorenzo Tessari (Tessari L., Cavezzi A., Frullini A., Preliminary experience with a new sclerosing foam in the treatment of varicose veins. Dermatol Surg 2001 Jan; 27 (1): 58-60) who published his experience using a foam easily manufactured through a procedure called "*The Tessari Method*". The method consists in the agitation of a sclerosing liquid using two syringes connected via a three-way tap. By means of successive alternately movements with each of the syringes connected to the gas/liquid mixture, a mix of foam, located at the inner part of the syringe, was achieved. However, this manufacturing technique, in spite of being the most widespread is not the most effective, since a relatively unstable and heterogeneous foam is obtained.

In recent years numerous articles and papers have been published about safety profiles, side effects and potential complications arising from the use of these products. Thus, it appears demonstrated that the best foam used is the one whose gas is a mixture of O₂/CO₂ at different concentrations. With this arrangement, the solubility and diffusion in blood is very high as opposed to atmospheric gas foams. Additionally, the foam stability is linked to the size of the bubble. Also, it has been found that the foam is more stable when the bubble diameter is more homogeneous.

As has already been mentioned, although there are a lot of manufacturing systems of sclerosing foam, the most common foam (and the one which is normally used around the world) is the foam obtained with the Tessari method. However, this method has many problems of standardization and homogenization. This system consists of mixing air flow with the selected liquid, either polidocanol or sodium tetradecyl sulfate (commercially known as sotradecol ®). This foam can have medium size bubbles, but of irregular size and it becomes unstable after a few seconds of its formation. Additionally, the use of atmospheric gas as a vehicle for the sclerosing foam limits the foam administrable per session.

The manufacture of different types of foams using commonly used medications and drugs results in great variability of foams. According to the manufacturing method used, and depending on the gas composition and the sclerosing agent, a foam of better or worse quality can be obtained.

There is therefore a need to find devices, kits, systems and methods for obtaining quality foams which are safe and can be obtained in a relatively cheap manner and without contamination.

### SUMMARY

In a first aspect, a container for the production of a foamed sclerosant composition is provided. The container comprises a sealed sterile container body for foaming having one or more sidewalls extending between a top and a bottom of the container body wherein a foaming space is defined in an interior of the container body. The container further comprises a mixing element disposed in the foaming space. The container contains a previously introduced gas and liquid sclerosant composition. The mixing element is configured to be operatively coupled with a rotable actuator without the actuator reaching the foaming space.

According to this first aspect, a sealed sterile container may be provided into which the gas and the sclerosant composition and a mixing element have been previously introduced. The three elements needed for obtaining a foam have therefore been introduced previously into the container in a secure industrial environment. The container body for foaming may be sterilized and packaged. According to this aspect, no introduction of an element from outside of the container is necessary after a medical professional has opened the packaging.

A mixing element is an element which ultimately creates a foam by mixing the contents of the container. Since the rotating actuator never enters into the foaming space, contamination of the foam can be reduced to a minimum or avoided completely. Activation of the actuator nevertheless causes the mixing element to rotate within the container thus producing a foam.

In some examples, the gas and the liquid sclerosant composition are contained in the foaming space. In these examples, the container may comprise a single container body wherein all three elements needed for the creation of the foam are already in the foaming space. In order to avoid any chemical reaction between e.g. the mixing element and the liquid sclerosant composition, the mixing element may be made from a substantially inert material.

In other examples, the container further comprises a sealed sterile drug container containing liquid sclerosant composition and the drug container is attachable to the container body. In these examples, the liquid sclerosant composition may be physically separated from the mixing element before use (i.e. during storage, transport etc.) of the container. Any influence of the mixing element on the stability or composition of the liquid sclerosant composition may be avoided. When a medical professional wants to prepare a sclerosant foam, the liquid sclerosant composition may be passed from the drug container to the foaming space.

In some of these examples, a container body for foaming and a drug container may be stored, and transported in a single packaging, but without being attached to each other. In other examples, during manufacture of the container, the container body for foaming and drug container may already be assembled. In either case, the resultant container may be sterilized and packaged.

A set or kit of different containers could be manufactured, sold and stored, wherein each of the container comprises the same container body (including a mixing element), but wherein the drug containers may vary in the amount of sclerosant liquid composition, and e.g. concentrations. Custom made containers could be provided for different types of treatment or different types of veins. The container body may be the same, but the contents of the drug containers can be adapted for different treatments and/or different veins. To facilitate the use for medical professional, the containers, and in particular drug containers for the different treatments and/or veins may have different colours, different labels (indicating type of treatment, vein, amount, concentration, physiological gas used etc.) or otherwise be visually distinguishable from one another.

In some examples, the drug container can be coupled to the container body allowing an axial movement during and/or after coupling. The axial movement (i.e. along the longitudinal axes of the drug container and container body) may bring the drug container closer to the container body. This axial movement can be used for bringing the liquid composition contained in the drug container into the container body for foaming. The axial movement may incorporate fracturing, rupturing, or breaking of a seal of the container body and/or the drug container. The seal may be e.g. a membrane or a lid or both. Many different sorts of coupling means, or coupling elements or coupling mechanisms may be used, and they may involve e.g. threads, helical threads, bayonet threads, or guides extending in an axial direction or snapping or clipping engagements involving e.g. elastic deformation of coupling elements or other.

In some examples, the drug container comprises first coupling means, and the container body comprises second coupling means that are adapted to mate with the first coupling means, and wherein the coupling means are configured for an axial movement of the drug container with respect to the container body during coupling. The coupling means may e.g. be threads or other means. In some examples, the axial movement from the drug container relative to the container body (i.e. this means that either the drug container moves or the container body moves, or both move), may cause the seal of the container body and drug container to be broken so that the sclerosant liquid composition may reach the mixing element. As may be seen in examples, this may be achieved while still preserving a sterile environment in the foaming space.

Achieving an operative coupling between the actuator outside the foaming space and the mixing element may be achieved in a variety of ways. In some examples, the mixing element may be mechanically coupled with the rotating actuator. In this case, the mechanical coupling may be such that the actuator couples with a portion of the mixing element outside the foaming space. For example, at a bottom of the container, there may be a suitably shaped notch in a proximal end of a rotating shaft extending upwards into the foaming space. An actuator may mate with the notch in order to establish an operative coupling between the actuator and the shaft.

In yet a further alternative, one or more mixing elements may be integrally formed with or attached to the container body, and the bottom of the container body may be configured to be mechanically coupled to the rotating actuator. In this case, foam could be generated simply by rotating the container body.

In other examples, a mixing element may be magnetically coupled with the rotating actuator. A magnetic coupling may be achieved by the mixing element comprising a magnetic portion or element, and the actuator as well. The magnetic coupling may be achieved through the walls of the container. The mixing element may be completely enclosed within the container body. The foaming space is thus not contaminated.

In yet further examples, the mixing element may be a disc with a plurality of blades around its circumference. This way, the contact surface between liquid, gas and the mixing element may be increased, thus the creation of the foam by mixing the contents of the container may also be improved.

In examples, in the preparation of a foamed sclerosant composition, physiological gases may be used. Such a physiological gas may be a mixture of CO₂ and O₂. For example, a mixture of 50/50 may be used. In some other examples, 70/30 may also be used (70% CO₂ and 30% O₂). A large proportion of CO₂ leads to quick absorption in the body. A large proportion of O₂ makes the foam more stable.

The use of physiological gases may be regarded as safer to the patient, than the use of (ambient) air. When large amounts of foam are to be injected, it is recommended to use physiological gases. The sterile containers according to these examples may thus be configured for their introduction.

In some examples, the container body may comprise a valve suitable for the extraction of the foamed sclerosant composition in the foaming space. Additionally or alternatively, the container body may comprise a frangible portion separable from the container body by a weakened region configured to facilitate the breaking of the sealed sterile container at the weakened region. A syringe may thus be inserted into the container body in a safe and clean manner, without contaminating the foam. The same syringe may subsequently be used for injection of the foamed sclerosant composition into a patient's veins. If a frangible portion of the container is used, the container has to be disposed of after a single use.

In a further aspect, a kit for the preparation of a foamed sclerosant composition is provided. The kit comprises a container according to any of the examples herein described, and further comprises a syringe for aspirating the foamed sclerosant composition.

In some examples, a kit may further comprise one or more drug container containing a liquid sclerosant composition.

Such kits may be packaged as a unit in a sterilized packaging.

In some examples, the drug containers comprising the liquid sclerosant composition may be squeezable drug containers. By squeezing of the container, it is made easier to introduce the liquid drug into the container without contamination.

In a further aspect, a method for preparing a foamed sclerosant composition is provided. The method comprises providing a container according to any of the examples herein described, and then rotating the actuator to rotate the mixing element until a suitable foam has been obtained.

In some examples, rotating the actuator may comprise rotating the actuator with a varying speed. During the foaming process, varying the speed, e.g. a gradual increase may be beneficial. For example, in cases wherein a magnetic coupling is used between actuator and mixing element, a gradual increase can ensure that the magnetic coupling is maintained and effective between actuator and mixing element.

In some examples, physiological gases may be used, in other examples ambient air may be used.

In yet a further aspect, a foamed sclerosant composition is provided, obtainable by any of the methods herein described. The foamed sclerosant composition as obtained may be distinguished from foams obtained by e.g. Tessari's method in its bubble size and homogeneity, and its stability.

Another aspect of the present disclosure relates to the foamed sclerosant composition obtainable by any of the methods herein described, for use in the treatment of varicose veins, spider veins, or haemorrhoids. Thus, this aspect relates to the use of a sclerosant drug, for the manufacture of a foamed sclerosant composition obtainable by any of the methods herein described, for the treatment of varicose veins, spider veins; and may also be formulated as a method for the treatment of varicose veins, spider veins, or haemorrhoids, which comprises administering a therapeutically effective amount of the foamed sclerosant composition obtainable by any of the methods herein described, in a subject in need thereof, including a human.

### BRIEF DESCRIPTION OF THE DRAWINGS

Particular implementations of the present disclosure will be described in the following by way of non-limiting examples, with reference to the appended drawings, in which:
Figures 1a - 1c schematically illustrate an example of a mixing element which may be used in a variety of containers for producing a foamed sclerosant composition;
Figures 2a - 2c schematically illustrate a container for preparing a foamed sclerosant composition and a sequence of steps in the preparation of a method for the production of a foamed sclerosant composition using the container according to an example;
Figures 3a - 3c schematically illustrates some further examples of a container for preparing a foamed sclerosant composition;
Figures 4a - 4e schematically illustrates a sequence of steps in the preparation of a method for the production of a foamed sclerosant composition using the container described in figures 3a - 3c; and
Figure 5 schematically illustrates another example of a container for preparing a foamed sclerosant composition.

### DETAILED DESCRIPTION

The expression "therapeutically effective amount" as used herein, refers to the amount of the foamed composition that, when administered, is sufficient to treat the diseases to which it is addressed. The specific dose which depends on both the volume and the drug concentration of the foamed sclerosant composition to obtain a therapeutic benefit may vary depending on the particular circumstances of each patient.

As previously mentioned, an aspect of the present disclosure relates to a foamed sclerosant composition obtainable by any of the methods herein described. The expression "obtainable by" is used herein for defining the foamed sclerosant composition by its preparation process. In particular, it refers to the foamed composition that can be obtained through the preparation process which comprises the previously commented steps.

For the purposes of the present disclosure, the expressions "obtainable", "obtained" and similar equivalent expressions are used interchangeably and, in any case, the expression "obtainable" encompasses the expression "obtained".

Throughout the present disclosure, the terms sclerosant and sclerosing are used interchangeably. Similarly, sclerosing foam, and foamed sclerosant composition are used interchangeably as well.

For the purposes of the present disclosure, the term foamed sclerosing composition refers to a composition of a foam capable of bringing about a sclerosing effect, i.e. a composition for use as a medicament for intravenous injection, which is capable of causing an injury to the vessel wall by endothelial vacuolation of the epithelial cell membrane (the layer in contact with the bloodstream). Thus, the foamed sclerosing composition irritates the inner surface of the vein just producing the formed thrombus formation by platelets and aggregates. Similarly, the term liquid sclerosing composition refers to a composition in liquid form including a sclerosing agent. The liquid sclerosing composition forms an ingredient to obtain the foamed sclerosing composition.

The sclerosing compositions according to examples of the present disclosure comprise a sclerosing agent and also a suitable vehicle which can be injected without toxicity in the affected veins. In some examples, the liquid is selected from sterile water (particularly distilled water) and physiological saline.

Examples of sclerosing agents that can be present in the sclerosing compositions of examples of the present disclosure include, without limitation, polidocanol, sodium tetradecyl sulfate, chromated glycerin, hypertonic saline, sodium morrhuate and sclerodex (hypertonic saline in combination with dextrose).

In a particular example, the sclerosing composition used comprises polidocanol and water. In another embodiment, the sclerosing composition further comprises glycerin.

In one particular example, the sclerosing composition may comprise a solution of a sclerosing agent, such as polidocanol, in a liquid, such as water or physiological saline, at a concentration from 2 mg to 20 mg in 1 mL liquid (which corresponds to 0.20 - 2.0 %) (w/v). In another example, the sclerosing composition may comprise a solution of a sclerosing agent, such as polidocanol, in a liquid, such as water or physiological saline, at a concentration from 2 mg to 5 mg in 1 mL liquid (which corresponds to 0.20 - 0.50 % (w/v)). With the devices and methods described herein, it has been found that even at very low concentrations e.g. 0.2% (w/v), still a stable foam may be obtained, contrary to e.g. Tessari's method.

In another particular example, the sclerosing composition may comprise a solution of polidocanol in water or physiological saline at a concentration of 5 mg/mL.

In another particular example, the sclerosing composition may comprise a solution of polidocanol in water or physiological saline at a concentration of 20 mg/mL.

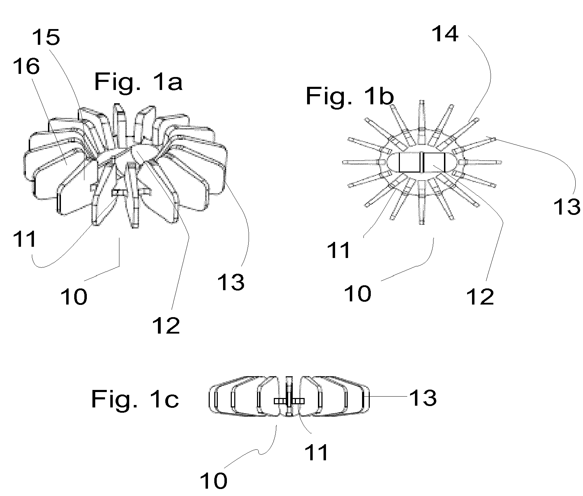

Figures 1a - 1c schematically illustrate an example of a mixing element which may be used in a variety of containers for producing a foamed sclerosant composition. Figure 1a schematically illustrates a three-dimensional view of a mixing element. The mixing element 10 in this example is substantially disc-shaped. The mixing element comprises a central opening or housing 12 in which a magnetic element (not shown) may be placed. In an example, the magnetic element or core may be kept in place between protrusions around the border of the central opening, shown in figure 1b. Figures 1b and 1c show respectively a top view and a side view of the same mixing element.

The disc or mixing element 10 in this example has a ring 11 with a plurality of vertically extending blades 13 along its circumference. Each of the blades comprises side faces. Adjacent blades e.g. blades 15 and 16 may be arranged with a gap between their side faces.

A housing for the magnetic element may be integrally formed with the blades or may be attached to the blades with e.g. adhesives or a friction or interference fit. In cases, wherein the magnetic element does not have a separate housing, it may be attached in similar ways. The magnetic element in such a case may comprise a protective layer that prevents any undesired chemical reaction between magnet and liquid.

The mixing element may be made of a non-magnetic material (apart from the magnetic core element). For example, a polymeric material may be used such as e.g. Polypropylene (PP) or Polytetrafluoroethylene (PTFE) or Nylon. In particular, non-magnetic materials that are substantially inert may be used. Inert herein means that the material is chemically not reactive.

With such an arrangement, the contact surface of the mixing element, particularly the side faces of the blades, with the gas and the liquid sclerosant composition may be increased. As the mixing element rotates, the gas and the liquid sclerosant passes through the channels or gaps between the side faces of the blades. An increased contact surface can improve the creation of the foamed sclerosant composition.

In some examples, instead of vertically extending blades, horizontally extending blades may be used. In some other examples, instead of a central opening, a central housing may be provided in which the magnetic core element is contained.

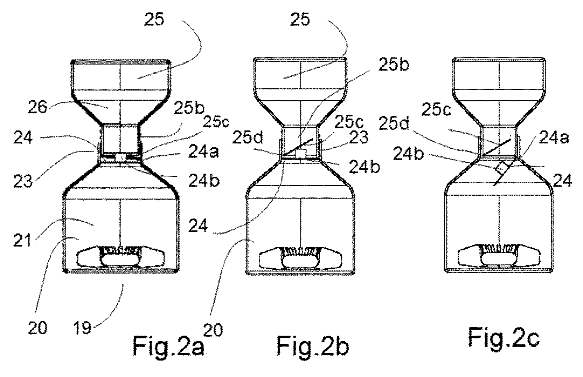

A mixing element such as the one shown in figures 1a - 1c may be used in the different examples of the containers shown in figures 2 - 5. In any of the examples, a magnetic stirrer may be used to set the mixing element in motion. The rotation of the mixing element causes mixing of the gas and liquid sclerosant composition to prepare a sclerosing foam.

Figures 2a - 2c schematically illustrate a container for preparing a foamed sclerosant composition and a sequence of steps in the preparation of a method for the production of a foamed sclerosant composition using the container according to an example. A mixing element according to the example of figures 1a - 1c might be used in combination with the container described below.

The container 19 in this example comprises a container body 20 for foaming. A foaming space 21 may be formed in the interior of the container body. In this particular example, the container body 20 may comprise a previously introduced physiological gas (a combination of O₂/CO₂ and between 30%-50% of O₂ and 70%-50% CO2). Due to the sterilization of the container body 20 for foaming and the quality control of the gas, the container body is virtually free of contamination. The container body 20 may be provided with a receiver 23. For the introduction of the liquid sclerosant, a drug container 25 with an introducer 25b may be provided. The introducer 25b and receiver 23 are complementary to each other, so that the introducer 25b and receiver maybe coupled together. The drug container 25 will be described in more detail below.

The receiver 23 in this example includes a cylindrical elongated portion with a drug container lid 24 closing off the drug container. The lid 24 is attached within the cylindrical portion of the receiver 23. Particularly, in this example, a first portion of an edge of the lid may be configured to be frangible or breakable when a pressure upon the lid is increased. A second portion 24a of an edge of the lid 24 along the circumference of the receiver is more durable, and when the first portion yields under the pressure, the second portion remains pivotally attached to the receiver 23. This way, the lid 24 may keep the container body 20 for foaming sealed in a first operational position. Reference may be had to figures 2a - 2b. In a second operational position, the lid 24 may be pivoted around the edge 24a due to the pressure exerted on the lid. Such pressure could be exerted by the liquid sclerosant derived from squeezing the drug container 25, thus the liquid sclerosant may be released into the container body 20. Reference may be had to figure 2c. In an example, a pressure is increased on the lid 24 by axially moving the drug container towards the container body for foaming, so that an edge of the drug container pushes against the lid. The lid 24 may further be provided with a protrusion 24b. The use and operation of the protrusion 24b will be described in more detail below.

As commented above, the sterile container 19 may comprise a drug container 25. The drug container may further comprise a hollow body 26 and an introducer 25b. The introducer 25b may be configured to mate with the complementary receiver 23. The introducer 25b (and thus the drug container) and the complementary receiver 23 (and thus the container body) may have a mechanical coupling e.g. a threaded coupling. The drug container 25 in this example is shown to be connected to the container body 20. The drug container and container body could be assembled, sterilized and packaged together, although it is also possible that they are sterilized and packaged and only assembled upon use. It is furthermore possible that drug container and container body for foaming are sterilized and packaged separately. It will be clear that there is no need for the exterior of the drug container to be sterilized. There would thus be no need for a sterile wrapping of the drug container.

Particularly in cases, wherein a vendor or manufacturer sells and packages drug containers separately from the container bodies for foaming, a medical professional may decide at the moment of assembly, which drug container to attach. A medical professional might then open the sterile packaging of the container body for foaming, attach the drug container, and empty the drug container in the container body (using any of the herein described methods for coupling / attaching of the drug container to the container body for foaming and any of the herein described methods for empyting the drug container). An actuator may be activated to rotate the mixing element and create the esclerosant foam.

Following the example, the drug container 25 may comprise a previously introduced liquid sclerosant composition. The drug container may be a squeezable drug container 25, thus the container may be squeezed in order to displace the liquid sclerosant composition within the container. The pressure exerted by squeezing the drug container 25 may result in the liquid sclerosant composition being dispensed from the drug container 25.

The drug container 25 may further comprise a lid 25c. Due to the sterilization of the drug container 25 and the quality control of the liquid sclerosant, the drug container 25 is virtually free of contamination. The lid 25c may be attached within the introducer. Particularly, a first portion of an edge of the lid may be configured to be broken (i.e. is frangible) under a given load, whereas another portion 25d of the edge of the lid 25c may withstand higher loads and remains pivotally attached to the introducer 25b. This way, the lid 25c may keep the drug container 25 sealed in a first operational position (see figure 2a). However, in a second operational position, wherein the lid 25c is pivoted, the liquid sclerosant may be released from the drug container 25 (see figures 2b - 2c).

When coupling the drug container and container body for foaming using the threads on introducer 25b and receiver 23, the introducer 25b is axially displaced relative to the receiver 23. As a result, the protrusion 24b of the lid can enter into contact and may impact with the lid 25c. Subsequently, this movement of the introducer 25b with respect to the receiver 23 may cause the stopper 25c to pivot about its edge 25d. Thus, the drug container may be opened, and the liquid sclerosant composition may flow out of the drug containers. It should be noted however that even in this situation, even though the drug container is opened, the sclerosant composition is still not exposed to the outside of the container and is still free from contamination.

In some of the experiments, a polymeric material container body of polypropylene has been used.

In accordance with an aspect, a sclerosant foam may be prepared substantially as follows:
Figure 2a illustrates an initial situation. The sterile container packaging is opened. In this initial situation, the introducer 25b (and thus the drug container 25) may be preassembled with the receiver 23 (and thus the container body 20). In other examples, a medical professional may perform the coupling at the moment of preparation of the foam or just before that moment.

The container body 20 for foaming contains a gas, as well as the rotatable disc with blades that carries at the centre a magnet as described in figures 1a - 1c. The gas may be a mixture of physiological gases e.g. 50/50 of CO₂ and O₂. Thus, the gas mixture chosen is maintained in the container body 20 for foaming and will be the gas contained in the bubbles of the foam. The drug container contains a liquid sclerosant composition.

In figure 2b, in order to release the liquid sclerosant composition into the container body 20 for foaming, the drug container 25 and the container body 20 are axially and rotatable displaceable with respect to each other. This way, the drug container 25 may rotate during the relative axial displacement. While rotating and during the relative axial displacement, the protrusion 24b may impact the lid 25c. Thus, the lid 25c may be pivoted about a portion 25d of its edge. Consequently, the liquid sclerosant composition may be released on the lid 24.

In figure 2c, the stopper 25c has been pivoted about its axis 25d, thus the liquid sclerosant composition has been released on the lid 24. As the drug container is screwed on further, pressure may build up on the lid 24, and as a result, the lid 24 may be pivoted about its edge 24a. This may result in the liquid sclerosant composition being released into the contained body. The liquid sclerosant composition may thus be inserted into the container body in a safe and clean manner, without contaminating the foam.

A magnetic actuator may be activated for rotating the mixing element and once the foam has been formed, the drug container 25 can be removed (for example, axially untwisted) and a syringe may be used for aspirating the foam.

Alternatively, the sterile container may be provided with a valve suitable for the extraction of the foamed sclerosant composition in the foaming space.

Once the foam has been formed, the foam may be extracted using an extraction area 35. The extraction area may comprise a suitable valve or e.g. a frangible portion.

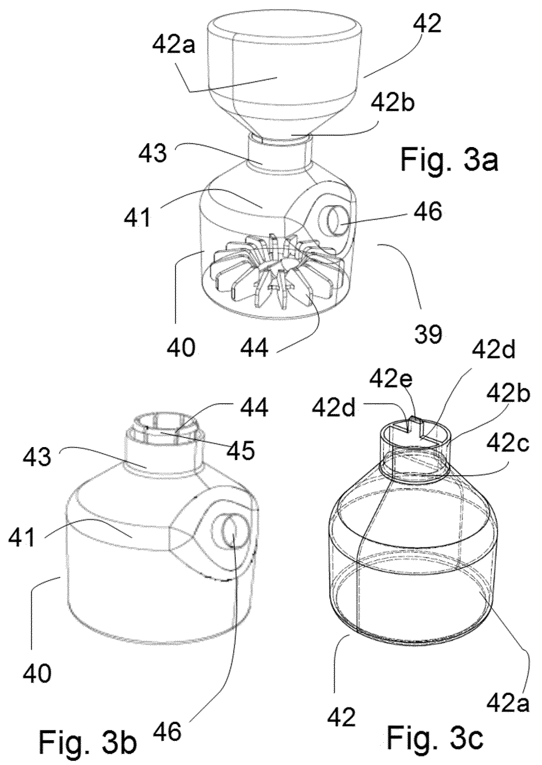

Figures 3a - 3c schematically illustrates yet another example of a container for preparing a foamed sclerosant composition. The mixing element 44 may be the same or substantially the same as previously described in figures 1a - 1 c.

Figure 3a schematically illustrates a three-dimensional view of a container. In this figure a sealed and sterile container 39 is provided. The container 39 may comprise a container body 40 for foaming. The container body 40 may contain a previously introduced gas e.g. physiological gas.

The container body 40 for foaming comprises a receiver 43. The receiver 43 may be suitable for the introduction of a liquid sclerosant. As shown in figure 3b, the receiver 43 in this example includes a cylindrical portion and a plurality of vertically extending fingers 44 radially offset from the cylindrical portion. At the ends of the fingers upstanding portions 45 or bulges may be provided. As the receiver may receive an introducer 42b of a drug container 42a, which will be explained in more detail below referring to figure 3c, the fingers may be pushed slightly inwards. Once the upstanding portions 45 extend beyond a cylindrical central extension of the introducer 42b, the upstanding portions, due to the elastic deformability of fingers 44, move outwards. A clipping or snapping engagement of introducer 42b with the receiver 43 may thus be achieved.

Additionally, the receiver 43 comprises a membrane (not shown). The container body for foaming is closed from the outside by the membrane. Therefore, the container body 40 for foaming (and thus the gas previously introduced) may be virtually free of contamination. This membrane may in some examples be provided with weakened regions. These regions are designed to facilitate the breaking of the sealed membrane at the weakened region.

The container body may further comprise a valve 46 for the extraction of the foam. The container body may be made of any suitable material e.g. glass, plastic, etc.

In figure 3c, a drug container 42 is shown comprising a hollow body 42a and an introducer 42b. The introducer 42b is configured to mate with the complementary receiver 43 of the sterile container, thus in use the drug container may be connected to the sterile container as shown in figure 3a. The engagement between the introducer and the receiver may be the same as previously described. The drug container 42 may be provided with a liquid sclerosant composition. The quality of the composition may be controlled, thus contamination in the composition can be avoided.

Additionally, the drug container 42 may be provided with a membrane 42c. The drug container 42 can thus be closed off from the outside by the membrane 42c. Again, this membrane may be provided with weakened regions. These regions are designed to facilitate the breaking of the sealed membrane at the weakened region.

The drug container 42 may further be provided with a central wall 42d. The central wall 42d may comprise a sharpened end or cutter 42e. The sharpened end 42e may be suitable for the perforation of the membrane of the receiver 43.

The container 39 may be sterilized and packaged in a sterile packaging, e.g. a wrap. Enclosed within the container a mixing element can already be provided. For example, mixing element of example 1 could be used.

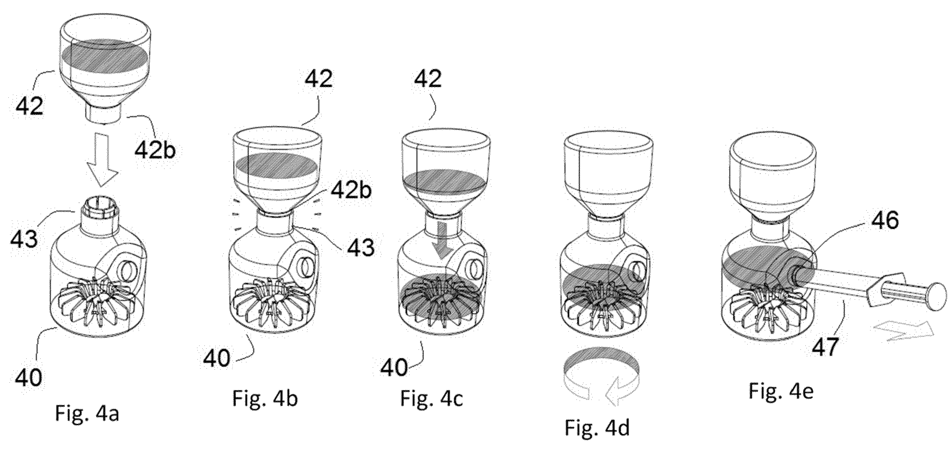

Figures 4a - 4e schematically sequence of situations occurring during the performance of a method for the production of a foamed sclerosant composition using the container described in figures 3a - 3c. Same reference numbers denote the same elements. The method is described below with reference to the sequences of situations illustrated by figures 4a - 4e.

The figure 4a illustrates an initial situation. In this initial situation, the drug container 42 and the container body 40 for foaming are shown. The drug container 42 may be previously provided with a liquid sclerosant composition. The container body 40 for foaming may be previously provided with a gas. The introducer 42b is ready to be introduced into the receiver 43 such that the liquid sclerosant may be released into the container body 40.

In figure 4b, the introducer 42b of the drug container 42 may be brought near the receiver 43 of the container body 40 for foaming. Once situated at the desired position, the introducer 42b may be advanced with a relative axial displacement relative to the receiver 43. As commented before the receiver may be provided with a plurality vertically extending fingers (shown in figure 4b). At the end of each finger upstanding portions may also be provided. As the receiver 43 may receive the introducer 42b of the drug container, the fingers may be pushed slightly inwards. Once the upstanding portions extend beyond a cylindrical central extension of the introducer 42b, the upstanding portions, due to the elastic deformability of fingers, move outwards. A clipping engagement of the introducer 42b and the receiver 43 may thus be achieved.

The wall 42d is adapted to fit in between diametrically opposite pairs of fingers so as to slide in between neighbouring fingers.

Additionally, while the introducer 42b advances relative to the receiver 43, the fingers can be used to puncture (and thus break) the drugs container's membrane. Furthermore, the sharpened end of the central wall may also be used to puncture (and thus break) the container body's membrane. In both membranes, the puncture may be performed at the weakened regions, thus the break of the membrane may be facilitated. With such an arrangement, the hermetic seal of the container body and the drug container may be broken. Thus, fluid communication is possible between drug container 42 and the container body 40.

In figure 4c, the drug container 42 has been properly clipped to the container body 40. Moreover, the container body's membrane and the drugs container's membrane have been punctured (and thus broken). At this stage, the drug container 42 may be squeezed. By squeezing of the drug container, it is made easier to introduce the liquid drug into the container body without contamination.

In figure 4d, the liquid sclerosant element has already been introduced into the container body 40 with virtually no contamination. At this stage, the mixing element may be activated, thus the whipping or the emulsification may be started

In figure 4e, once the foam has been formed, a syringe may be used for aspirating the foam. This may be performed by introducing a syringe though the valve 46. It may be a one-way valve.

In alternative examples, the cutter or sharpened end may be provided on the container body, whereas the fingers are provided on the drug container. A substantially similar coupling may be achieved.

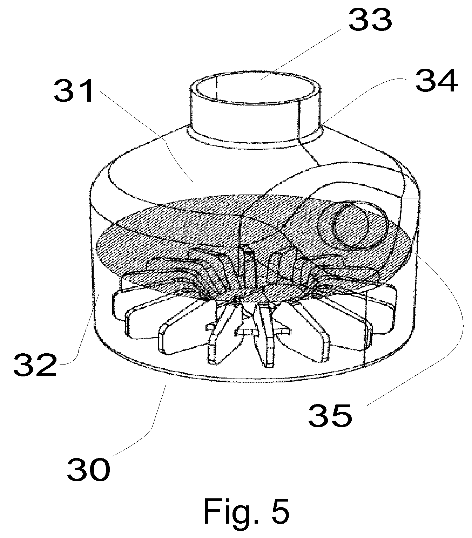

Figure 5 schematically illustrates another example of a container for preparing a foamed sclerosant composition. A container 30 is shown. A mixing element may be provided. The structure and operation of the mixing element may be the same as previously described in figures 1a - 1c. The container 30 may further be provided within a foaming space 31. The foaming space 31 may be formed in the interior of the container body 32. In this particular example, the foaming space 31 may also contain a previously introduced gas e.g. physiological gas and a previously introduced liquid sclerosant composition. The container 30 itself may be of generally one-piece molded plastic construction. Although it may be made of any suitable material, medically inert plastic, vinyl, polyethylene or polypropylene, glass are typical materials which may be used.

In this example, in a single container body, mixing element, gas and liquid sclerosant composition are provided. The container body in this case is both the body for foaming and for storing the liquid sclerosant composition. In this example, there is no need for a separate drug container. The container as shown may be sterilized and packaged e.g. in a wrap and stored as such. The materials for the mixing element and container body may be chosen such that stability and composition of the gas and liquid can be maintained.

The sterile container may also be provided with a valve 35 suitable for the extraction of the foamed sclerosant composition in the foaming space.

Thus, a syringe (not shown) may be inserted into the container body using the valve 35 or the frangible portion 33 in a safe and clean manner, without contaminating the foam. The same syringe may subsequently be used for injection of the foamed sclerosant composition into a patient's veins. If a frangible portion 33 of the container is used, the container has to be disposed of after a single use.

Also in this example, the rotating actuator does not enter in the interior of the container body, i.e. in the foaming space. The foaming space is sealed from the outside. The foaming space (and the foam once is created) may thus be virtually free of contamination.

In accordance with an aspect, a sclerosant foam may be prepared substantially as follows:
The sterile container packaging may be opened. At this stage, the container 30, particularly the container body 32, may contain a previously introduced physiological gas and liquid sclerosant composition therein, as well as the rotatable mixing element. In order to make the foam, contamination is avoided since the quality of the liquid sclerosant composition and the gas is previously controlled. Additionally, the container body is sterilized.

The mixing element may be activated, thus the whipping or the emulsification may be started.

In any of the illustrated examples, a standard magnetic stirrer which is frequently found in laboratories may be used as a rotating actuator. The container 19 may be positioned on the magnetic stirrer which may comprise clamps to hold the container. The magnetic stirrer when running causes a rotating magnetic field. The rotating field may be created either by a rotating magnet or a set of stationary electromagnets. The rotating magnetic field drags along the magnetic core of the mixing disc and thereby sets it into rotation.

Experiments have been carried out using a magnetic stirrer with an analogic control type AGIMATIC® (code 7000242) without heating for speeds of 60 to 1600 rpm. It may comprise an upper plate of stainless steel (type 304 AISI®) having a diameter of 14.5 cm, wherein the container can be positioned. It also has a security ring against spilling which consists of two 15 cm diameter plastic discs having a central opening of 5 cm, wherein the container can be placed.

The system can use sterile atmospheric air which is contained inside the container body 20 or a gas mixture based on a physiological composition based in a combination of O₂ / CO₂. Depending on the varicose vein to be treated, depending on the profile of each patient and/or injected desired volume, it can be decided to use a container containing either air or a physiological gas mixture.

In some examples of the containers as described throughout the present disclosure, the container might comprise a one-way valve for the introduction of a gas. This may simplify manufacture in that ambient air is easier to use than a mixture of physiological gases.

This treatment can be applied to a large variety of types of varicosity, of size and of location, on an outpatient basis and without limitation in performing daily activities. From spider veins, varicose veins or capillaries subcutaneous veins to large volume varicose veins, practically all of them can be treated by the resulting foam.

Another aspect offered with a sclerotherapy technique performed with the foam in examples of the present disclosure may be that no fasting or any specific preparation of the patient is required. The patients who follow the treatment with anticoagulants therapy such as aspirin, clopidogrel or similar or with oral acenocoumarol type (Sintrom®) may not be required to suspend their treatment.

The rotating actuator does not enter in the interior of the container body, i.e. in the foaming space. The foaming space is sealed from the outside. The foaming space may thus be virtually free of contamination.

It has been found that it can be advantageous to increase the rotational speed of the magnetic stirrer gradually. Generally within two or three minutes enough stable foam has been formed.

In general, rotational speeds between 300 RPM and 1.800 RPM have been tested and shown to work well. At higher rotational speeds, a foam may be formed quicker. But if the magnetic stirrer rotates too fast, the magnetic coupling between stirrer and mixing element can be lost. For this reason, a gradual increase of rotation speed can be beneficial.

In some examples, mixing is performed during 30 seconds - 4 minutes, more particularly during 1 minute - 3 minutes, and preferably between 1 - 2 minutes.

Furthermore, in any of the shown examples, different containers could be provided containing different compositions of physiological gas, different quantities of liquid sclerosant composition and at different concentrations. A medical profession could thus select the most suitable container or containers depending on the needs of a patient.

For reasons of completeness, various aspects of the present disclosure are set out in the following numbered clauses:
Clause 1. A container for the production of a foamed sclerosant composition, the container comprising:
   - a sealed sterile container body for foaming having one or more sidewalls extending between a top and a bottom of the container body, a foaming space being defined in an interior of the container body
   - a mixing element disposed in the foaming space, and
      wherein the container further contains a previously introduced:
      ∘ gas, and
      ∘ a liquid sclerosant composition.
      wherein the mixing element is configured to be operatively coupled with a rotatable actuator without the actuator entering into the foaming space.
Clause 2. A container according to clause 1, wherein the gas and the liquid sclerosant composition are contained in the foaming space.
Clause 3. A container according to clause 1, further comprising a sealed sterile drug container wherein the liquid sclerosant composition is contained in a sealed sterile drug container, the drug container being attachable to the container body for foaming.
Clause 4. A container according to clause 3, wherein the drug container is attached to the container body for foaming.
Clause 5. A container according to clause 4, in a sterile packaging.
Clause 6. A container according to any of clauses 3 - 5, wherein the drug container comprises first coupling means, and the container body for foaming comprises second coupling means that are adapted to mate with the first coupling means, and wherein the coupling means are configured for an axial movement of the drug container with respect to the container body for foaming during coupling and/or after coupling.
Clause 7. A container according to clause 6, wherein the first and second coupling means are mating threads.
Clause 8. A container according to clause 5, wherein the second coupling means comprise a plurality of upstanding fingers, the fingers having bulges near their proximal ends, and wherein the first coupling means are configured to mate with the fingers by snapping over the bulges.
Clause 9. A container according to any of clauses 6 - 8, wherein the container body for foaming comprises a container body lid for closing off the container body and the drug container comprises a drug container lid for closing off the drug container, and wherein the container body lid and/or the drug container lid are configured to be opened in the axial movement of the drug container relative to the container body.
Clause 10. A container according to clause 9, wherein the container body lid comprises a membrane and the drug container lid comprises a cutter for perforating the membrane in the axial movement of the drug container relative to the container body for foaming.
Clause 11. A container according to clauses 8 and 9, wherein the drug container lid comprises a membrane, and wherein the fingers are configured to perforate the membrane.
Clause 12. A container according to clause 9, wherein the drug container lid is frangible along a portion of an edge of the drug container lid and is pivotable along at least another portion of the edge of the drug container lid, the container body lid is frangible along a portion of an edge of the container body lid and is pivotable along at least another portion of the edge of the container body lid.
Clause 13. A container according to clause 12, wherein the container body lid comprises a central upward protrusion.
Clause 14. A container according to any of clauses 1 - 12, wherein the gas is a physiological gas, and optionally is a mixture of O₂ and CO₂.
Clause 15. A container according clause 14, wherein a percentage of 02 is between 30% - 50%.
Clause 16. A container according to any of clauses 1 - 15, wherein the container body for foaming further comprises a frangible portion in sidewall or in a bottom of the container body.
Clause 17. A container according to clause 16, wherein the frangible portion is located at the bottom of the container body.
Clause 18. A container according to clause 16, wherein the frangible portion is in a sidewall near the bottom of the container body.
Clause 19. A container according to any of clauses 1 - 15, wherein the container body for foaming comprises a valve suitable for the extraction of the foamed sclerosant composition in the foaming space.
Clause 20. A container according to clause 19, wherein the valve is in the sidewall of the container body for foaming.
Clause 21. A container according to any of clauses 3 - 20 wherein the drug container is a squeezable drug container.
Clause 22. A container according to any of clauses 1 - 21, wherein the container body for foaming is made of glass.
Clause 24. A container according to any of clauses 1 - 21, wherein the container body for foaming is made of plastic.
Clause 25. A container according to any of clauses 1 - 24, comprising a mixing shaft having a proximal end and a distal end, wherein
   the distal end is configured to be coupled with the mixing element
   the proximal end is configured with the rotatable actuator, and wherein
   the mixing element is optionally integrally formed with the mixing shaft.
Clause 26. A container according to any of clauses 1 - 24, wherein one or more mixing elements are integrally formed with or attached to the container body, and the bottom of the container body is configured to be mechanically coupled to the rotating actuator.
Clause 27. A container according to any of clauses 1 - 24, wherein the mixing element comprises a magnetic element, and wherein the rotatable actuator is configured to cause a rotating magnetic field, and optionally is a magnetic stirrer.
Clause 28. A container according to clause 27, wherein the container body is made from a non-magnetic material.
Clause 29. A container according to clause 27 or 28, wherein the mixing element is made from an inert material.
Clause 30. A container according to any of clauses 1 - 29, wherein the mixing element is a disc with a plurality of blades around its circumference.
Clause 31. A container according to any of clauses 1 - 30, wherein the liquid sclerosant composition is a solution of the drug in distilled water or a saline.
Clause 32. A container according to any of clauses 1 - 30 enclosed in a sterile packaging.
Clause 33. A container according to any of clauses 1 - 32, wherein the volume ratio liquid / gas in the foaming space is between 1 / 4 and 1 / 12, and optionally between 1/6 and 1/9.
Clause 34. A kit for the preparation of a foamed sclerosant composition comprising a container according to any of clauses 1 - 33, further comprising a syringe for aspirating the foamed sclerosant composition prepared in the sterile container.
Clause 35. A system for the production of a foamed sclerosant composition comprising a sterile container according to any of clauses 1 - 33 and an actuator configured to be operatively coupled to the mixing element.
Clause 36. A method for preparing a foamed sclerosant composition comprising:
   - providing a container according to any of clauses 1 - 33;
   - activating the actuator to rotate the mixing element until a suitable foam has been obtained.
Clause 37. A method according to clause 36, further comprising attaching the drug container to the container body for foaming and introducing the sclerosant liquid composition in the foaming space before activating the actuator.
Clause 38. A method according to clause 36 or 37, further comprising aspirating the foamed sclerosant composition.
Clause 39. A method according to any of clauses 36 - 38, wherein activating the actuator comprises rotating the actuator with a varying speed and wherein the actuator optionally is a magnetic stirrer and the speed of rotation is between 60 - 1.800 RPM, and optionally between 300 - 1.800 RPM.
Clause 40. A method according to any of clauses 36 - 39, comprising activating the actuator for 30 seconds - 5 minutes, preferably 45 seconds - 3 minutes.
Clause 41. A method according to any of clauses 36 - 40, wherein the liquid sclerosant composition is a solution of a sclerosant drug in water, optionally distilled water, or a saline.
Clause 42. A method according to clause 41, wherein a concentration of the drug is 0,20 - 2.0 % (w/v).
Clause 43. A method according to clause 41, wherein the concentration of the drug is 0,20 - 0,50 % (w/v).
Clause 44. A method according to any of clauses 36 - 43, wherein the previously introduced liquid sclerosant composition comprises between 2 - 10 ml, and optionally about 5 ml of liquid sclerosant composition.
Clause 45. A foamed sclerosant composition obtainable by a method according to any of clauses 36 - 44.
Clause 46. A foamed sclerosant composition according to clause 45, for use in the treatment of spider veins.
Clause 47. A foamed sclerosant composition according to clause 45, for use in the treatment of haemorrhoids.

Although only a number of particular embodiments and examples of the invention have been disclosed herein, it will be understood by those skilled in the art that other alternative embodiments and/or uses of the invention and obvious modifications and equivalents thereof are possible. Furthermore, the present invention covers all possible combinations of the particular embodiments described.

In particular, combinations of aspects of various examples are possible in the sense that, different methods of activation of the mixing element (mechanical or magnetic) may be used in different containers, as well as different shapes and sizes of mixing elements. Furthermore, the methods of coupling of the drug container and container body and the methods for introduction of the liquid sclerosant into the drug container are independent from the amount of liquid, the concentration of the drug, the type of gas used, and indeed the type of mixing element used.

Thus, the scope of the present invention should not be limited by particular embodiments, but should be determined only by a fair reading of the claims that follow.

## Claims

1. A container for the production of a foamed sclerosant composition, the container comprising:
- a sealed sterile container body for foaming having one or more sidewalls extending between a top and a bottom of the container body, a foaming space being defined in an interior of the container body
- a mixing element disposed in the foaming space, and
wherein the container further contains a previously introduced:
∘ gas, and
∘ a liquid sclerosant composition.
wherein the mixing element is configured to be operatively coupled with a rotatable actuator without the actuator entering into the foaming space.

2. A container according to claim 1, further comprising a sealed sterile drug container, wherein the liquid sclerosant composition is contained in the drug container, the drug container being attachable to the container body for foaming.

3. A container according to claim 2, wherein the drug container is attached to the container body for foaming.

4. A container according to claim 3 in a sterile packaging.

5. A container according to any of claims 2 - 4, wherein the drug container comprises first coupling means, and the container body for foaming comprises second coupling means that are adapted to mate with the first coupling means, and wherein the coupling means are configured for an axial movement of the drug container with respect to the container body for foaming during coupling.

6. A container according to claim 5, wherein the container body comprises a container body lid for closing off the container body for foaming and the drug container comprises a drug container lid for closing off the drug container, and wherein the container body lid and/or the drug container lid are configured to be opened in the axial movement of the drug container relative to the container body for foaming.

7. A container according to claim 6, wherein the container body lid comprises a membrane and the drug container lid comprises a cutter for perforating the membrane in the axial movement of the drug container relative to the container body for foaming.

8. A container according to claim 6, wherein the drug container lid is frangible along a portion of an edge of the drug container lid and is pivotable along at least another portion of the edge of the drug container lid, the container body lid is frangible along a portion of an edge of the container body lid and is pivotable along at least another portion of the edge of the container body lid.

9. A container according to claim 5, wherein the second coupling means comprise a plurality of upstanding fingers, the fingers having bulges near their proximal ends, and wherein the first coupling means are configured to mate with the fingers by snapping over the bulges.

10. A container according to any of claims 2 - 9 wherein the drug container is a squeezable drug container.

11. A container according to claim 1, wherein the gas and the liquid sclerosant composition are contained in the foaming space.

12. A container according to any of claims 1 - 11, wherein the gas is a physiological gas, and optionally is a mixture of O₂ and CO₂, and wherein a percentage of O₂ is between 30% - 50%.

13. A container according to any of claims 1 - 12, wherein the container body for foaming further comprises an extraction area for extracting a foam, wherein the extraction area optionally comprises a frangible portion in sidewall or in a bottom of the container body.

14. A container according to any of claims 1 - 13, wherein the mixing element comprises a magnetic element, and wherein the rotating actuator is configured to cause a rotating magnetic field, and optionally is a magnetic stirrer.

15. A container according to any of claims 1 - 14, wherein the volume ratio liquid / gas in the foaming space is between 1 / 4 and 1 / 9.
